# EUROPEAN PATENT APPLICATION

(11) **EP 1 676 830 A1**
(43) Date of publication of application: **05.07.2006**
(21) Application number: 05027385.3
(22) Date of filing: 14.12.2005
(51) Int. Cl.: C07C 67/37, C07C 69/738, B01J 23/44, B01J 31/22

(54) **Carbonylation of alpha-chloroketones to beta-keto esters using palladium carbene catalysts**

(30) Priority: 30.12.2004 US 27260
(71) Applicant: EASTMAN CHEMICAL COMPANY, Kingsport, TN 37660 (US)
(72) Inventor: Zoeller, Josef Robert, Kingsport Tennessee 37660 (US)
(74) Representative: Wibbelmann, Jobst

(57) **Abstract**

The present invention is a novel process for producing β-ketoesters **1** by contacting an α-chloroketone **2** with carbon monoxide and a hydroxyl-containing compound of formula R³OH in the presence of a base and a palladium carbene catalyst complex **3**. The subject catalysts demonstrate superior rates and selectivity when compared to known (Ph₃P)₂PdCl₂.

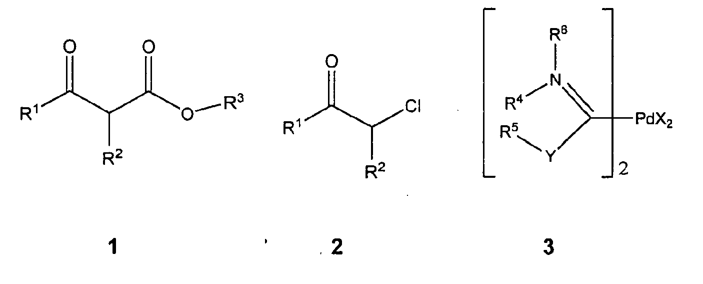

## Description

### Field of the Invention

The present invention is a novel process for producing a β-ketoester by contacting an α-chloroketone with carbon monoxide and a hydroxyl-containing compound of formula R³OH in the presence of a base and a palladium carbene catalyst complex.

### Backaround of the Invention

β-Ketoesters (such as in figure **1**, below) are widely used chemical intermediates to generate a wide array of specialty chemicals, such as pharmaceuticals, pesticides, pigments, and dietary supplements. A classical method used to generate these compounds includes a Claisen condensation in which two esters are condensed in the presence of one or more equivalents of an expensive strong base such as sodium hydride, or a modified process wherein a ketone is condensed with a carbonate ester. Another traditional method is a Blaise reaction in which an α-bromocarboxylic acid ester is reacted with a nitrile in the presence of one or more equivalents of zinc. The Claisen and related condensation processes suffer from the need for a stoichiometric amount of an expensive strong base, and normally demonstrate poor yields when using dissimilar esters. The Blaise process suffers from the expense of nitriles, usually gives mediocre yields, and generates a large waste stream because it generates a stoichiometric Zn and nitrogen (ammonium) salt by-product that must be sent to an appropriate disposal or treatment facility.

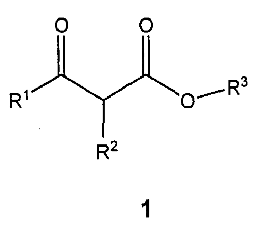

Given the drawbacks associated with such classical methods, industrial production often is accomplished by other means. The simplest β-ketoesters, the acetoacetates, are available by reacting diketene with an alcohol. Diketene is toxic and unstable and is not a common item of commerce. Producers normally generate diketene by the high temperature pyrolysis of acetic acid or acetone to generate ketene, and then allow the ketene to dimerize to diketene. The diketene is later reacted with an alcohol on site to generate an acetoacetate ester, which is then offered for sale. Industrially, these simple acetoacetates are then used to generate more complex β-ketoesters (e.g., by subsequent functionalization or acyl group exchange).

For example, Rathke, et. al., *J. Org. Chem*., **50**, 2622 (1985) demonstrated that simple acetoacetates can exchange their acetyl group for more complex acyl groups by reacting them with acyl halides in the presence of stoichiometric amounts of magnesium halide salts and pyridine derivatives according to the equation below: 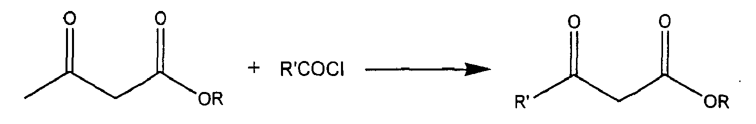 Although the original process required stoichiometric amounts of a magnesium salt, a subsequent process, catalytic in magnesium, has been demonstrated, although the only example disclosed was for generating pivaloylacetate esters (R' = tert-butyl) from pivaloyl chloride and aceotacetate esters. (See, e.g., U. S. Patent 6,570,035.)

Further substitution at the α position is normally accomplished by subsequent reaction of the aceotacetates formed by the means described above. It is well known to those practitioners skilled in the art that alkylation at the α position can be induced by adding a base and an organic halide or pseudohalide, but it is preferred, especially if mono substitution is the preferred product, to condense the acetoacetate with an aldehyde or ketone and hydrogenate the product since the condensation and hydrogenation are both catalytic and selective and there is no by-product halide salt generated in the process. Normally the condensation and hydrogenation are conducted simultaneously in the same vessel.

More recently, a promising method for directly generating β-ketoesters involves carbonylating α-chloroketones in the presence of an alcohol using a (Ph₃P)₂PdCl₂ catalyst. (Lapidus et al., *Russian Chemical Bulletin, Int. Edit.,* **50**, 2239 (2001); Lapidus et al., *Synthesis,* 317 (2002).) The α-chloroketones used therein are either commercially available or can often be generated by chlorinating the parent ketone. (See U. S. Patent 4,186,144.)

Palladium catalysts and their complexes are known in the art. One particular type of palladium complex is known as a carbene complex. (See below, for example.) For reviews of metal carbene complexes, see for example, Hermann et al., *Advances in Organometallic Chemistry,* **48,** 1 (2001); Hermann, *Angew. Chem., Int. Ed.,* **41,** 1290 (2002); Hermann et al., *Angew. Chem. Int. Ed.,* **36,** 2162 (1997); or Hermann et al., *Chem. Eur. J.*, **2,** 772 (1996). Specific examples of palladium carbene complexes may be found in, for example, Hermann et al., *Angew. Chem. Int Ed.,* **34,** 2371 (1995); Hermann et al., US Pat. 5,703,269 (1997); Caddick et al., *J. Organomet. Chem.,* **617-618,** 635 (2001); Hermann et al. J. *Organomet. Chem.,* **617-618**, 616 (2001); Viciu et al., *Organometallics,* **22,** 3175 (2003); Viciu et al., *Org. Lett.,* **4**, 2229 (2002); Viciu et al., *Org. Lett.,* **4**, 4053 (2002); Glas et al., *J. Organomet. Chem.,* **626,** 100 (2001); Lewis et al., *J. Amer. Chem. Soc*., **125,** 10066 (2003);or Furstner et al., *Organometallics,* **22***,* 907 (2003).

While such palladium complexes are known and have been used in catalysis, their application in a carbonylation appears to have been limited to a carbonylation of aromatic halides using the carbene catalyst below. (See Calo et al., *J. Organomet. Chem.,* **645**, 152 (2002).) The use of palladium carbenes for generating β-ketoesters appears to be heretofore unknown.

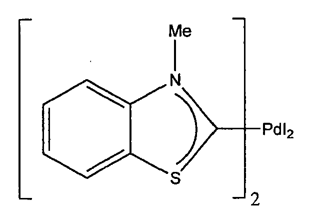

### Brief Summary of the Invention

The present invention is a process for producing β-ketoesters of formula **1** 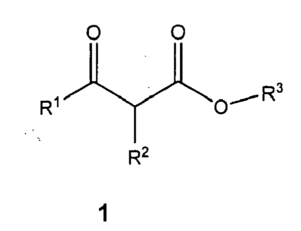 which comprises contacting an α-chloroketone of formula **2** 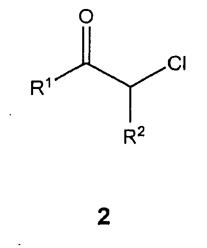 with carbon monoxide and a hydroxyl-containing compound of formula R³OH in the presence of a base and a catalyst of formula **3** 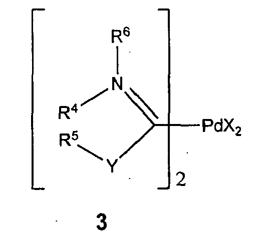 wherein R¹ through R⁶, X and Y are as set forth below. In addition, the present invention relates to a novel carbonylation catalyst comprising a palladium carbene catalyst complex of the general formula **3** above, in which Y is N-alkyl or N-aryl.

### Detailed Description

As stated above, the present invention, as exemplified below in equation [**A**], is a process for generating β-ketoesters of formula **1** 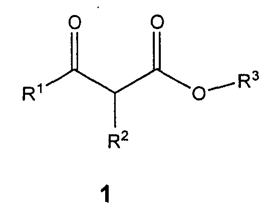 which comprises contacting an α-chloroketone of formula **2** 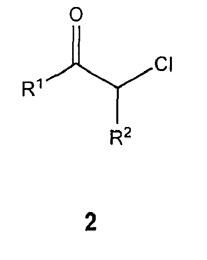 with carbon monoxide and a hydroxyl-containing compound of formula R³OH in the presence of a base and a catalyst having formula **3** 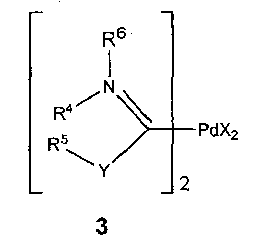 In the present invention, R¹ and R² are, independently, hydrogen, C₁ - C₁₀ alkyl, C₃ - C₁₀ cycloalkyl, C₆ - C₂₀ aryl, C₁ - C₁₀ alkenyl, or a C₁ - C₁₀ hetero-alkyl, a C₁ - C₁₀ heteroalkenyl, or a C₄ - C₁₀ heteroaryl group containing, in addition to the carbon content, up to three heteroatoms selected from O, S, or N, or R¹ and R² may be joined together with any of the foregoing groups to form a bridging group (e.g., R¹ and R² may represent a C₁ - C₁₀ alkylene bridge). R³ may be C₁ - C₁₀ alkyl, C₃ - C₁₀ cycloalkyl, C₆ - C₂₀ aryl, C₁ - C₁₀ alkenyl, or a heteroalkyl or heteroaryl as above. R⁴, R⁵ and R⁶ are, independently, C₁ - C₂₀ alkyl, C₃ - C₁₀ cycloalkyl, C₆ - C₂₀ aryl, C₁ - C₁₀ alkenyl, or a C₁ - C₁₀ heteroalkyl, a C₁ - C₁₀ heteroalkenyl, or a C₄ - C₁₀ heteroaryl group containing, in addition to the carbon content, up to three heteroatoms selected from O, S or N, or R⁴ and R⁵, and optionally R⁶, may be joined together with any of the foregoing groups to form a bridging group (e.g., a bridging alkylene of up to 10 carbons). Y is C₁ - C₂₀ alkyl-N, C₃ - C₁₀ cycloalkyl-N, C₆ - C₂₀ aryl-N, heteroalkyl-N, heteroaryl-N, O, or S; and X is an anion. All substituents, R¹ - R⁶, may contain additional functional groups such as, but not limited to, C₁ - C₂₀ alkoxy, C₁ - C₂₀ aryloxy, C₁ - C₂₀ carboxyl, C₁ - C₂₀ amino, C₁ - C₂₀ alkylamino, C₁ - C₂₀ amido, or C₁ - C₂₀ thioalkyl.

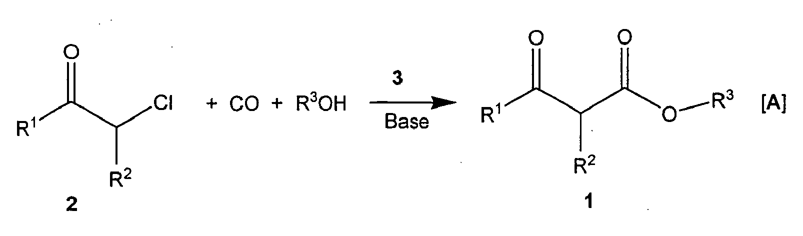

The α-chloroketone starting material, and thus the resulting β-ketoester, may be selected from any of a wide class of compounds, as described above. The inventive process is particularly useful for generating the class of β-ketoesters in which R¹ is C₆ - C₁₀ aryl or C₁ - C₁₀ alkyl or C₃ - C₁₀ cycloalkyl, in which any of the foregoing groups may optionally be substituted with a variety of substituents, including, but not limited to halogen (e.g., fluorine, chlorine, or bromine), C₁ - C₂₀ alkoxy, C₁ - C₂₀ aryloxy, C₁ - C₂₀ carboxyl, C₁ - C₂₀ amino, C₁ - C₂₀ alkylamino, C₁ - C₂₀ amido, or C₁ - C₂₀ thioalkyl. Particularly useful are processes in which R₁ is *tert*-butyl, and R₂ is hydrogen. The resulting compounds are useful in a wide variety of applications, but their synthesis by traditional means is difficult.

The hydroxyl-containing compound for use in the invention may be any of a wide variety of substances encompassed by the description above. The hydroxyl-containing compound is preferably described by R³OH, in which R³ is a C₁ - C₁₀ alkyl group; in particular, R³ may be a methyl group (C₁) or an ethyl group (C₂) (e.g., methanol or ethanol). The alcohol reactant for use herein may also function as a process solvent.

The reaction of the present invention generates an equivalent of HCl from the chloroketone starting material. Thus, as noted, the process of the present invention employs a base. Although the base could in theory be chosen from any of a host of basic alkaline earth and alkali metal salts to scavenge the HCl, such bases likely give poor selectivities. The bases useful herein are those in the group of organic nitrogen-containing bases (amines). The amine base may be an alkyl amine, such as a trialkyl amine or a heterocyclic aromatic amine, such as pyridine and substituted pyridines. It is most convenient to employ trialkyl amines wherein each alkyl group is a C₁ - C₁₀ alkyl, C₃ - C₁₀ cycloalkyl or C₆ - C₁₀ aryl; examples include triethylamine, tripropylamine, tributylamine, di-isopropyl ethyl amine and trioctyl amine.

The ratio of base:chloroketone for use herein can be in the range of 10:1 to 1:1, with improved performance in the range of 2:1 to 1:1, and better performance in the range of 1.25:1 to 1.75:1. The foregoing trialkyl amine bases, and their salts, are readily soluble in the reaction medium, or can be readily dissolved upon warming or the addition of small amounts of alcohol, yet can be easily separated from the product mixture and recovered for further use by extraction and neutralization.

As stated above, I have now found that palladium carbene complexes having general formula **3** below, are superior catalysts for carbonylating α-chloroketones to the corresponding β-ketoesters. Processes using such palladium carbene complexes demonstrate both higher rates and higher selectivities to the desired β-ketoesters as compared to processes using known (Ph₃P)₂PdCl₂ catalysts. The catalyst has the general formula **3** 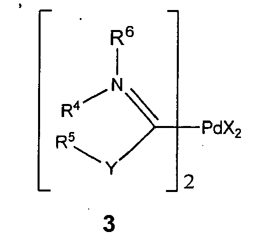 wherein R⁴, R⁵ and R⁶ are, independently, C₁ - C₂₀ alkyl, C₃ - C₁₀ cycloalkyl, C₆ - C₂₀ aryl, C₁ - C₁₀ alkenyl, C₁ - C₁₀ heteroalkyl, C₁- C₁₀ heteroalkenyl, or C₄ - C₁₀ heteroaryl group containing, in addition to the carbon content, up to three heteroatoms selected from O, S or N, or R⁴ and R⁵, and optionally R⁶, may be joined together with any of the foregoing groups to form a bridging group (e.g., a bridging alkylene of up to 10 carbons); Y is C₁ - C₂₀ alkyl-N, C₃-C₁₀ cycloalkyl-N, C₆ - C₂₀ aryl-N, heteroalkyl-N, heteroaryl-N, O, or S; and X is an anion. All of substituents R⁴ through R⁶ may contain further functional groups such as, but not limited to, C₁ - C₂₀ alkoxy, C₁ - C₂₀ aryloxy, C₁ - C₂₀ carboxyl, C₁ - C₂₀ amino, C₁ - C₂₀ alkylamino, C₁ - C₂₀ amido, or C₁ - C₂₀ thioalkyl. Further, the present invention relates to a novel carbonylation catalyst comprising a palladium carbene catalyst complex of the general formula **3** above, in which Y is C₁ - C₂₀ N-alkyl or C₆ - C₂₀ N-aryl.

The substitutions on the palladium carbene catalyst complex **3** may be selected from any number of groups or moieties described above, but R⁴ and R⁵ most conveniently are, or include a 2 carbon bridging group, such as 1,2-substituted phenyl, 1,2-substitued ethylenyl (e.g., -HC=CH-), or 1,2 substituted-ethanyl (e.g., -CH₂CH₂-). R⁶ may be a number of substituents, but is normally a C₁ - C₁₀ alkyl group or a C₆ - C₂₀ aryl group, such as a 2,6-dialkyl substituted aryl group such as di-isopropyl (e.g., 2,6-diisopropyl phenyl) or 2,4,6- trisubstitutied such as 2,4,6-trimethyl phenyl (often called by its common name mesityl).

Y may be a sulfur or an oxygen as noted above, but is more commonly a nitrogen containing group, such as N-R⁷, in which R⁷ is a C₁ - C₂₀ alkyl group or a C₆ - C₂₀ aryl group, such as a 2,6-dialkyl substituted aryl group such as a 2,6-dialkyl substituted aryl group such as di-isopropyl (e.g., 2,6-diisopropyl phenyl) or 2,4,6- trisubstitutied such as 2,4,6-trimethyl phenyl. While R⁶ and R⁷ may be the same or different groups, most commonly the resulting catalysts are symmetrical, with R⁶ and R⁷ being identical. X may be any of a number of anions, including tetrafluoroborate, carboxylate or acetate, but is most commonly and conveniently a halide, such as iodide, bromide, or chloride.

Methods for generating catalysts described herein are well known to those of skill in the art. A convenient method for generating such catalysts is one in which a palladium carboxylate, preferably palladium acetate, reacts with a halide salt of a quaternary salt such as is set forth in formula **4** below, in a solvent. The solvent may be any easily removed solvent, but is usually an organic oxygenate such as a simple ester or ether, such as tetrahydrofuran or ethyl acetate. The groups R⁴, R⁵, R⁶, X, and Y are as described above. Once reacted, the solvent is removed and the catalyst can then be purified by very simple chromatographic means. Methods for preparing catalyst precursor **4** are well known to those of skill in the art, or a number of such precursors corresponding to **4** can be purchased commercially.

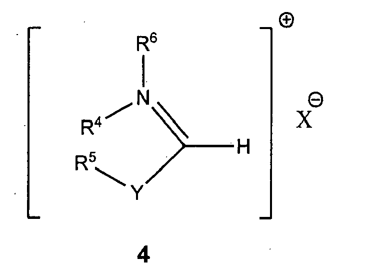

Although it is common practice to generate the subject catalysts *in situ* from a quaternary salt, such as described by **4**, and a palladium salt when using such catalysts in other catalytic applications, this practice normally leads to inferior catalyst performance in this application. Therefore, it is preferred to generate the catalyst externally and then use the preformed catalyst in the catalytic process.

The reaction is normally operated in a solvent that can be selected from a wide array of organic solvents, including but not limited amides, ethers, alcohols, esters, aromatic hydrocarbons, but the preferred solvent is the alcohol corresponding to R³ of the product. For example, if the desired product is a methyl ester, the preferred solvent would be methanol, and if it were an ethyl ester, it would be ethanol.

While operable under ambient pressure and temperature conditions, the process is normally performed under conditions of elevated temperature and pressure. The operable pressure range is from 0.1 to 100 atmospheres absolute pressure (atm) (or 0.01 to 10 MPa), with the preferred pressure range being 5 to 20 atm (0.5 to MPa). The temperature can be from 0°C to 250°C; the preferred range is 75°C to 175°C, and more preferably 100°C to 150°C.

The operable pressure for the reaction is in the range of 1 to 100 atmospheres (atm) absolute pressure (0.1 to 10 MPa absolute pressure). Normally, the process is operated in the range of 3 to 50 atm (0.3 to 5 MPa) absolute pressure. As the skilled artisan will appreciate, optimal pressure is a complex function of temperature and the nature and concentration of the reaction components, particularly the choice and concentration of alcohol and trialkyl amine, since these variables significantly affect the vapor pressure exerted by the reaction mixture. However, the preferred pressure range is 5 to 35 atm (0.5 to 3.5 MPa) absolute pressure.

The skilled artisan will understand that each of the references herein to groups or moieties having a stated range of carbon atoms, such as "C₁-C₁₀-alkyl," includes not only the C₁ group (methyl) and C₁₀ group (decyl) end points, but also each of the corresponding individual C₂, C₃, C₄, C₅ and so forth, groups. In addition, it will be understood that each of the individual points within a stated range of carbon atoms may be further combined to describe subranges that are inherently within the stated overall range. For example, the term "C₁-C₁₀-alkyl" includes not only the individual moieties C₁ through C₁₀, but also contemplates subranges such as "C₂-C₅-alkyl."

This invention can be further illustrated by the following examples of preferred embodiments thereof, although it will be understood that these examples are included merely for purposes of illustration and are not intended to limit the scope of the invention.

### Examples

*Catalyst Preparation.* The following synthesis of catalyst **5a** was typical. To a 100 mL round bottom flask was added 736 mg (1.74 mmol) of N,N-bis-(2,6-diisopropylphenyl) imidazolium chloride, 192 mg (0.85 mmol) of palladium acetate and 50 mL of dry, degassed tetrahydrofuran. The mixture was heated to reflux and reflux maintained for 4 hours. The mixture was cooled, the solvent reduced to about ½ the original volume on a rotary evaporator and the mixture was then filtered through a cylindrical silica gel column measuring 5.0 cm in width and about 3.5 cm in height using 10% ethyl acetate in hexane to elute the catalyst from the silica gel. Removal of solvent provides 602 mg (0.629 mmol, 74% yield) of dichloro-bis (N, N'-bis - (2,6-di-isopropyl phenyl) immidazol-1-ylidene) palladium (II) (**5a**) as a mixture of cis and trans isomers and used without further purification. Catalysts **5b** , **5c**, and **5d** were prepared from palladium acetate and the corresponding imidazolium halide in a similar manner in 54%, 50%, and 67% yields respectively. 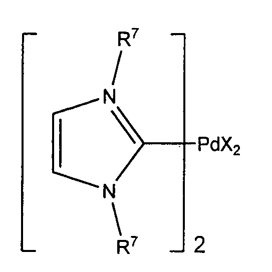
- **5a,** R⁷ =: 2,6-diisopropylphenyl-, X = Cl
- **5b,** R⁷ =: 2,4,6-trimethylphenyl-, X = Cl
- **5c,** R⁷: = methyl, X = I
- **5d**, R⁷: = n-Bu, X = Br

### Example 1

To a 300 mL Hastelloy® C autoclave was added 110 mL of methanol, 13.1 mL (0.1 mol) of 1-chloropinacolone (1-chloro-3,3,-dimethyl-1-chloro-2-butanone), and 36 mL (0.15 mol) of tributyl amine followed by 95.4 mg (0.1 mmol) of **5a**. The mixture was sealed, flushed thoroughly with carbon monoxide, and then pressurized to a gauge pressure of 2 atm (0.2 MPa). The mixture was heated to 120 C and the pressure adusted to a gauge pressure of 10 atm (1.0 MPa) with carbon monoxide. The temperature and pressure were maintained for 3 hrs using carbon monoxide as needed to maintain the pressure. The autoclave was cooled and depressurized. The product was analyzed for pinacolone (3,3-dimethyl-2-butanone), 1-chloropinacolone, and methyl pivaloylacetate (methyl 4,4-dimethyl-3-oxo-1-pentanoate) using gas chromatography and found to contain 0.12 wt% pinacolone, 2.25 wt% chloropinacolone, and 8.76 wt% methyl pivaloyl acetate. This corresponds to a chloropinacolone conversion of 78% with a selectivity of 93% toward methyl pivaloylacetate and only 2.0% loss to pinacolone. The palladium turnover frequency was 241 mol methyl pivaloyl acetate produced /mol Pd/hr.

### Comparative Example A

Example 1 was repeated except that 70.1 mg (0.1 mmol) of dichloro-bis-triphenylphospine palladium (II), which is the catalyst used in analogous examples in the art [see, A. L. Lapidus, et. al. *Russian Chemical Bulletin, Int. Edit.,* **50**, 2239 (2001); A. L. Lapidus, *et. al., Synthesis,* 317 (2002)] was substituted for catalyst **5a**. Upon analysis by gas chromatography, the mixture was found to contain 0.34 wt% pinacolone, 3.68 wt% chloropinacolone, and 5.14 wt% methyl pivaloyl acetate. This corresponds to a chloropinacolone conversion of 64% with a selectivity of 65% toward methyl pivaloylacetate and a 6.8% loss to pinacolone. The palladium turnover frequency was 140 mol methyl pivaloyl acetate produced /mol Pd/hr.

Comparative Example A demonstrates that the reaction with a known catalyst is inferior in selectivity (giving less of the desired methyl pivaloylacetate and more of the undesired pinacolone per unit of consumed starting 1-chloropinacolone) and rate (generating less methyl pivaloyl acetate and demonstrating a lower turnover frequency) when compared to the catalyst of the present invention under the same conditions.

### Example 2

Example 1 was repeated except that 78.6 mg (0.1 mmol) of catalyst **5b** was substituted for catalyst **5a.** Upon analysis by gas chromatography, the mixture was found to contain 0.14 wt% pinacolone, 3.90 wt% chloropinacolone, and 6.69 wt% methyl pivaloyl acetate. This corresponds to a chloropinacolone conversion of 62% with a selectivity of 89% toward methyl pivaloylacetate and a 2.9% loss to pinacolone. The palladium turnover frequency was 183 mol methyl pivaloyl acetate produced /mol Pd/hr.

### Example 3

Example 1 was repeated except that 55.4 mg (0.1 mmol) of catalyst **5c** was substituted for catalyst **5a**. Upon analysis by gas chromatography, the mixture was found to contain 0.54 wt% pinacolone, 3.56 wt% chloropinacolone, and 5.96 wt% methyl pivaloyl acetate. This corresponds to a chloropinacolone conversion of 66% with a selectivity of 75% toward methyl pivaloylacetate and a 10.7% loss to pinacolone. The palladium turnover frequency was 183 mol methyl pivaloyl acetate produced /mol Pd/hr.

### Example 4

Example 1 was repeated except that 62.5 mg (0.1 mmol) of catalyst **5d** was substituted for catalyst 5a. Upon analysis by gas chromatography, the mixture was found to contain 0.11 wt% pinacolone, 3.70 wt% chloropinacolone, and 5.03 wt% methyl pivaloyl acetate. This corresponds to a chloropinacolone conversion of 64% with a selectivity of 64% toward methyl pivaloylacetate and a 2.2% loss to pinacolone. The palladium turnover frequency was 137 mol methyl pivaloyl acetate produced /mol Pd/hr.

### Example 5

To a 300 mL Hastelloy® C autoclave was added 110 mL of ethanol, 15.3 g (0.1 mol) of 1-chloroacetophenone, and 36 mL (0.15 mol) of tributyl amine followed by 95.4 mg (0.1 mmol) of **5a**. The mixture was sealed, flushed thoroughly with carbon monoxide, and then pressurized to a gauge pressure of 2 atm (0.2 MPa). The mixture was heated to 105°C and the pressure adjusted to a gauge pressure of 10 atm (1.0 MPa) with carbon monoxide. The temperature and pressure were maintained for 3 hrs using carbon monoxide as needed to maintain the pressure. The autoclave was cooled and depressurized. The product was analyzed for acetophenone, 1-chloroacetophenone (starting material) and ethyl benzoylacetate (3-phenyl-3-oxo-1-propanoate) using high pressure liquid chromatography and found to contain 0.31 wt.% acetophenone, 0.56 wt% chloroacetophenone and 10.94 wt% methyl pivaloyl acetate. This corresponds to a chloropinacolone conversion of 95% with a selectivity of 80% toward ethyl benzoylacetate and a 3.4% selectivity loss to acetophenone. The palladium turnover frequency was 253 mol ethyl benzoylacetate produced /mol Pd/hr.

### Comparative Example B

Example 5 was repeated except that 70.1 mg (0.1 mmol) of dichloro-bis-triphenylphospine palladium (II), which is the catalyst used in analogous examples in the art, was substituted for catalyst **5a**. Upon analysis by high pressure liquid chromatography the product was found to contain 0.28 wt% acetophenone, 0.03 wt% chloroacetophenone and 8.17 wt% methyl pivaloyl acetate. This corresponds to a chloropinacolone conversion of 99.7% with a selectivity of only 57% toward ethyl benzoylacetate and a 3.2% selectivity loss to acetophenone. The palladium turnover frequency was 201 mol ethyl benzoylacetate produced /mol Pd/hr.

As in Comparative Example A, the foregoing Comparative Example B demonstrates that the catalyst of the present invention shows higher selectivities and higher rates toward the desired product than a known catalyst when used in the carbonylation of 1-chloroacetophenone.

## Claims

1. A process for generating β-ketoesters of formula 1 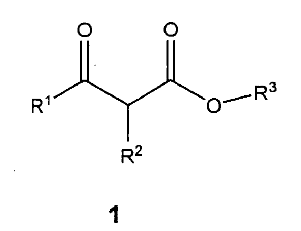 which comprises contacting an α-chloroketone of formula **2** 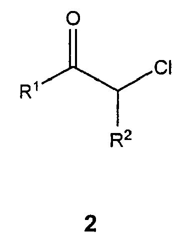 with carbon monoxide and R³OH in the presence of a base and a catalyst having formula 3 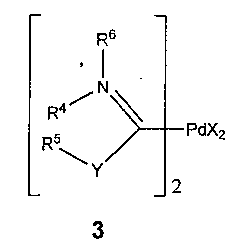 wherein R¹ and R² are, independently, hydrogen, C₁ - C₁₀ alkyl, C₃ - C₁₀ cycloalkyl, C₆ - C₂₀ aryl, C₁ - C₁₀ alkenyl, C₁ - C₁₀ heteroalkyl, C₁ - C₁₀ heteroalkenyl, or C₄ - C₁₀ heteroaryl containing, in addition to the carbon content, up to three heteroatoms selected from O, S, or N, or R¹ and R² may be joined together with any of the foregoing groups to form a bridging group; R³ is C₁ - C₁₀ alkyl, C₃ - C₁₀ cycloalkyl, C₆ - C₂₀ aryl, C₁ - C₁₀ alkenyl, C₁ - C₁₀ heteroalkyl or C₄ - C₁₀ heteroaryl containing, in addition to the carbon content, up to three heteroatoms selected from O, S or N; R⁴, R⁵ and R⁶ are, independently, C₁ - C₂₀ alkyl, C₃ - C₁₀ cycloalkyl, C₆ - C₂₀ aryl, C₁ - C₁₀ alkenyl, C₁ - C₁₀ heteroalkyl, C₁ - C₁₀ heteroalkenyl, or C₄ - C₁₀ heteroaryl containing, in addition to the carbon content, up to three heteroatoms selected from O, S or N, or R⁴ and R⁵, and optionally R⁶, may be joined together with any of the foregoing groups to form a bridging group; Y is C₁ - C₂₀ alkyl-N, C₃ - C₁₀ cycloalkyl-N, C₆ - C₂₀ aryl-N, heteroalkyl-N, heteroaryl-N, O, or S; wherein all substituents for R¹ - R⁶ may be substituted with C₁ - C₂₀ alkoxy, C₁ - C₂₀ aryloxy, C₁ - C₂₀ carboxyl, C₁ - C₂₀ amino, C₁ - C₂₀ alkylamino, C₁ - C₂₀ amido, or C₁ - C₂₀ thioalkyl; and X is an anion.

2. A process as claimed in claim 1 wherein R¹ is C₆ - C₂₀ aryl, C₃ - C₁₀ cycloalkyl, or C₁ - C₁₀ alkyl each of which may optionally be further substituted with halogen, a C₁ - C₂₀ alkoxy, C₁ - C₂₀ aryloxy, C₁ - C₂₀ carboxyl, C₁ - C₂₀ amino, C₁ - C₂₀ alkylamino, C₁ - C₂₀ amido, or C₁ - C₂₀ thioalkyl; R² is hydrogen; R³ is C₁ - C₁₀ alkyl; R⁴ and R⁵ are collectively a bridging group; R⁶ is C₁ - C₂₀ alkyl, C₃ - C₁₀ cycloalkyl, or C₆ - C₂₀ aryl; X is chloride, iodide or bromide; and Y is N-R⁷ wherein R⁷ is C₁ - C₂₀ alkyl, C₃ - C₁₀ cycloalkyl, or C₆ - C₂₀ aryl.

3. A process as claimed in claim 2 wherein R¹ is *tert*-butyl; R³ is methyl or ethyl; R⁴ and R⁵ are collectively 1,2-substituted phenyl, 1,2-substituted ethylene, or 1,2-substituted ethane; X is chloride; and R⁶ and R⁷ are each a 2,6- or 2,4,6-dialkyl substituted aryl group.

4. A process according to claim 3 wherein and R⁶ and R⁷ are, independently 2,6-di-isopropyl phenyl or 2,4,6-trimethyl phenyl.

5. A process as claimed in claim 2 wherein the contacting is performed at a pressure of 0.1 to 100 atmospheres absolute pressure (atm), and at a temperature of 75°C to 175°C.

6. A process as claimed in claim 5 wherein the contacting is performed at a pressure of 5 to 20 atm and at a temperature of 100°C to 150°C.

7. A process for generating β-ketoesters of formula 1 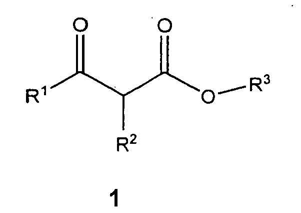 which comprises contacting an α-chloroketone of formula 2 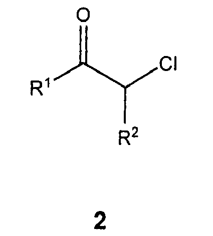 with carbon monoxide and R³OH in the presence of a base and a catalyst having formula 3 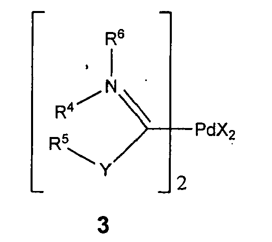 wherein R¹ is C₆ - C₂₀ aryl, C₃ - C₁₀ cycloalkyl or C₁ - C₁₀ alkyl each of which may optionally be substituted with halogen, C₁ - C₂₀ alkoxy, C₁ - C₂₀ aryloxy, C₁ - C₂₀ carboxyl, C₁ - C₂₀ amino, C₁ - C₂₀ alkylamino, C₁ - C₂₀ amido, or C₁ - C₂₀ thioalkyl; R₂ is hydrogen; R³ is C₁ - C₁₀ alkyl; R⁴ and R⁵ are collectively a bridging group; R⁶ is C₁ - C₂₀ alkyl or C₆ - C₂₀ aryl group; X is chloride, iodide or bromide; Y is N-R⁷ wherein R⁷ is C₁ - C₂₀ alkyl or C₆ - C₂₀ aryl and the contacting is performed at a pressure of 0.1 to 100 atmospheres absolute pressure (atm), and at a temperature of 75°C to 175°C.

8. A process as claimed in claim 7 wherein R¹ is *tert*-butyl; R³ is methyl or ethyl; R⁴ and R⁵ are collectively 1,2-substituted phenyl, 1,2-substituted ethylene, or 1,2-disubstituted ethane; X is chloride; and R⁶ and R⁷ are each a 2,6- or 2,4,6-dialkyl substituted aryl group.

9. A process as claimed in claim 8 wherein the contacting is performed at a pressure of 5 to 20 atm and at a temperature of 100°C to 150°C.

10. A palladium carbene carbonylation catalyst of formula 3 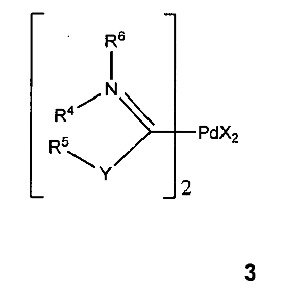 wherein R⁴, R⁵ and R⁶ are, independently, C₁ - C₂₀ alkyl, C₃ - C₁₀ cycloalkyl, C₆ - C₂₀ aryl, C₁ - C₁₀ alkenyl, C₁ - C₁₀ heteroalkyl, C₁ - C₁₀ heteroalkenyl, or C₄ - C₁₀ heteroaryl containing, in addition to the carbon content, up to three heteroatoms selected from O, S or N, or R⁴ and R⁵, and optionally R⁶, may be joined together with any of the foregoing groups to form a bridging group; Y is N-R⁷ wherein R⁷ is C₁ - C₂₀ alkyl or C₆ - C₂₀ aryl; and X is chloride, iodide or bromide.

11. A catalyst as claimed in claim 10 wherein R⁴ and R⁵ are collectively a bridging group; R⁶ and R⁷ are, independently, C₁ - C₂₀ alkyl or C₆ - C₂₀ aryl; and X is chloride.

12. A catalyst as claimed in claim 11 wherein R⁴ and R⁵ are collectively 1,2-substituted phenyl, 1,2-substituted ethylene, or 1,2-substituted ethane; X is chloride; and R⁶ and R⁷ are each a 2,6- or 2,4,6-dialkyl substituted aryl group.

13. A process as claimed in claim 12 wherein R⁶ and R⁷ are each independently, 2,6-di-isopropyl phenyl or 2,4,6-trimethyl phenyl.
